# EUROPÄISCHE PATENTSCHRIFT

(11) **EP 4 244 169 B1**
(45) Veröffentlichungstag und Bekanntmachung des Hinweises auf die Patenterteilung: **09.09.2026**
(21) Anmeldenummer: 21810337.2
(22) Anmeldetag: 10.11.2021
(51) Int. Cl.: B66B 11/02

(54) **AUFZUGSKABINE**
LIFT CAR
CABINE D'ASCENSEUR

(30) Priorität: 12.11.2020 EP 20207300
(43) Veröffentlichungstag der Anmeldung: 20.09.2023
(73) Patentinhaber: INVENTIO AG, 6052 Hergiswil (CH)
(72) Erfinder: CARRARA, Elena, 6005 Luzern (CH); LIEBETRAU, Christoph, 5737 Menziken (CH); SCHMITT, Dominik, 6037 Root (CH)
(74) Vertreter: Inventio AG
(86) Internationale Anmeldenummer: PCT/EP2021/081206
(87) Internationale Veröffentlichungsnummer: WO 2022/101250

(56) Entgegenhaltungen:
- CN-A- 111 573 456
- CN-A- 111 821 479
- DE-U1- 202017 100 914

## Beschreibung

Die Erfindung betrifft eine Aufzugskabine mit einer UVC-Strahlungseinrichtung. Aufzugsanlagen zur Beförderung von Personen und Gütern enthalten Aufzugskabinen, die in einem Aufzugsschacht auf und ab bewegbar sind. Die Kabinen können über Tragmittel, beispielsweise in Form von Tragseilen oder Tragriemen, mittels einer Antriebseinheit bewegt werden. Häufig weisen Aufzugskabinen Kabinenbedienpanel zum Eingeben eines Zielstockwerks auf, welche vom Aufzugsnutzer über Fingerberührung bedient werden. Dadurch können gegebenenfalls gefährliche Krankheitskeime wie aktuell Covid-19-Viren auf das Kabinenbedienpanel gelangen und anderen Aufzugsnutzern übertragen werden. Es besteht daher das Bedürfnis, Kabinenbedienpanel oder andere Eingabeeinrichtungen in der Kabine zu desinfizieren.

Aus der DE 20 2017 100914 U1 ist eine gattungsmässig vergleichbare Aufzugskabine mit einer UVC-Strahlungseinrichtung bekannt geworden. So lange keine Menschen in das Innere der Aufzugskabine eintreten, desinfizieren die von den UV-Elementen erzeugten UV-Strahlen die Raumluft und Oberfläche, beispielsweise eine Drucktastentafel und ein Geländer der Aufzugskabine.

Es ist eine Aufgabe der vorliegenden Erfindung, die Nachteile des Bekannten zu vermeiden und insbesondere eine Aufzugskabine zu schaffen, mit der auf einfache und effiziente Art und Weise Eingabeeinrichtungen in der Kabine desinfiziert werden können.

Diese und andere Aufgaben werden erfindungsgemäss mit einer Aufzugskabine mit den Merkmalen des Anspruchs 1 gelöst. Die Aufzugskabine weist wenigstens eine an einer ersten Kabinenseitenwand angeordnete Eingabeeinrichtung zum Generieren eines Steuersignals an die Aufzugssteuerung oder an eine andere elektronische Vorrichtung auf. Die Eingabeeinrichtung kann insbesondere ein Kabinenbedienpanel zum Eingeben eines Zielstockwerks sein, beispielsweise eine Tastatur zum Eingeben einer wählbaren Zielstockwerknummer. Das Kabinenbedienpanel kann zusätzlich oder unter Umständen auch alternativ Tasten zum Eingeben eines Schliessbefehls zum Schliessen der Türe oder Offenhaltebefehl, eine Notruftaste, etc. enthalten. Die Eingabeeinrichtung kann zum Beispiel auch eine Vorrichtung zur Vermittlung von Informationen für Aufzugsbenutzer sein. Solche Informationen können Informationen zu Wetter, Sportresultaten, Sportveranstaltungen, Börsendaten, Unterhaltung, Comics, etc. sein. Über die Eingabeeinrichtung kann dem Aufzugsnutzer je nach persönlichem Interesse Information mittels einer Anzeigeeinrichtung zur Verfügung gestellt werden. Vorstellbar ist auch, dass über die Eingabeeinrichtung aufzugsvertrauten Personen aufzugsspezifische Information zur Verfügung gestellt werden. Alternativ oder zusätzlich kann das Aufzugs--Fachpersonal über die Eingabeeinrichtung mit dem Aufzug kommunizieren und statistische Information und/oder Betriebsdaten abfragen. Die Eingabeeinrichtung kann zum Beispiel Tasten oder Knöpfe enthalten, die durch Drücken betätigt werden. Bekannt und gebräuchlich sind mechanisch arbeitende elektrische Tasten, kapazitive Tasten oder Piezotasten. Die Eingabeeinrichtung kann auch andere manuelle Eingabemittel enthalten. Zum Beispiel könnte die Eingabeeinrichtung auch ein Bildschirm mit berührungssensitiven Eingabebereichen (Touchscreens) umfassen.

Die in der Regel quaderförmige Aufzugskabine kann einen durch Kabinenseitenwände, eine Kabinendecke und einem Kabinenboden vorgegebenen Kabineninnenraum aufweisen. Die Aufzugskabine weist eine UVC-Strahlungseinrichtung zum Bestrahlen der Eingabeeinrichtung mit Strahlung im UVC-Bereich zum Desinfizieren der von Aufzugsnutzern berührten Oberflächen der Eingabeeinrichtung auf, welche Strahlungseinrichtung an der Kabinendecke angeordnet ist. Wenn die Eingabeeinrichtung an einer ersten Kabinenseitenwand angeordnet ist, dann ist UVC-Strahlungseinrichtung bevorzugt in einem hinteren Bereich in der Nähe bzw. benachbart einer der ersten Kabinenseitenwand gegenüberliegenden zweiten Kabinenseitenwand angeordnet. Die erste Kabinenseitenwand und die zweite Kabinenseitenwand sind dabei in einer Längsrichtung voneinander beabstandet. Die die erste Kabinenseitenwand und die zweite Kabinenseitenwand miteinander verbindenden Seitenwände verlaufen folglich jeweils in die erwähnte Längsrichtung. Eventuell kontaminierte Oberflächen auf der Eingabeeinrichtung können dank der günstig in oder an der Kabinendecke positionierten UVC-Strahlungseinrichtung mittels Strahlung im UVC-Bereich zwischen 200 und 300nm desinfiziert werden. In diesen Bereichen absorbieren die Keime, genauer gesagt deren DNA das UV-Licht und es kommt zu einer dauerhaften Schädigung der DNA/RNA. Diese Schädigung führt wiederum dazu, dass sich der Keim nicht vermehren kann und abstirbt. Unerwünschte Bakterien, Legionellen, Viren, Hefen und Pilze auf der Eingabeeinrichtung können so zuverlässig unschädlich gemacht werden. Bevorzugt ist ein Wellenlängenbereich zwischen 250nm und 280nm, in welchem eine besonders wirksame Desinfektion sichergestellt werden kann. Die Desinfektionswirkung unter Verwendung von UVC-LEDs kann bei niedrigem Energieverbrauch erfolgen. UVC-LEDs verfügen weiterhin über eine lange Lebensdauer und sind sehr verschleissarm.

Die erste Kabinenseitenwand kann ein an eine Kabinentüre anschliessender vertikaler Wandabschnitt sein. Dieser Wandabschnitt und damit die erste Kabinenseitenwand würde somit auf derselben Seite wie die Kabinentüre liegen. Die erste Kabinenseitenwand kann aber auch eine Kabinenseitenwand sein, die vorzugsweise rechtwinklig zum türseitigen Wandabschnitt verläuft. Es wäre aber auch denkbar, dass die erste Kabinenseitenwand die Rückseite der Aufzugskabine bildet, welche Rückseite der durch die Kabinentür definierten Vorderseite der Aufzugskabine gegenüberliegt.

Für eine effektive Bestrahlung der Eingabeeinrichtung umfasst die UVC-Strahlungseinrichtung eine Vielzahl von in horizontaler Richtung nebeneinander angeordneten UVC-LEDs als Strahlungsquellen. Das Nebeneinander bezieht sich auf die Richtung, die quer zur vorgängig erwähnten Längsrichtung verläuft. Die nebeneinander angeordneten UVC-LEDs bilden eine Strahlungsquellen-Reihe, die sich in Querrichtung zur Längsrichtung erstreckt. Ein weiterer Vorteil dieser Anordnung besteht im geringen Platzbedarf der UVC-Strahlungseinrichtung.

Die jeweiligen UVC-LEDs können in einer punktweisen Anordnung nebeneinander in der UVC-Strahlungseinrichtung positioniert sein. Jeder Punkt repräsentiert eine Strahlungsquelle. Die einzelnen Punkte müssen jedoch keine Punkte im geometrischen Sinne sein. Die einzelnen UVC-LEDs bzw. Strahlungsquellen können auch eine Gruppe aus mehreren UVC-Strahlung emittierende Dioden sein. Die nebeneinander angeordneten UVC-LEDs können in regelmässigen Abständen aneinander gereiht sein, wobei der Abstand zwischen zwei UVC-LEDs beispielhaft mindestens 1 cm und vorzugsweise wenigstens 2 cm betragen kann. Es ist aber auch denkbar, die UVC-LEDs je Reihe aneinander anzuschliessen, so dass die UVC-LEDs eine linenförmige Anordnung bilden.

UVC-Bestrahlung ist problematisch, weil sie gesundheitsschädigend für die Augen und auch für die menschliche Haut ist. Daher ist die UVC-Strahlungseinrichtung bevorzugt derart ansteuerbar, dass gewährleistet ist, dass die Bestrahlung der Eingabeeinrichtung nur bei Abwesenheit von Personen in der Kabine möglich ist. Beispielsweise kann in der Aufzugskabine wenigstens ein Sensor vorgesehen sein, mit dessen Hilfe feststellbar ist, ob Personen in der Aufzugskabine anwesend sind oder diese betreten. Für die Detektion von Personen kann ein solcher Sensor beispielsweise einen Bewegungsmelder, einen Lichtvorhang, eine Videokamera, eine Lichtschranke, einen Photosensor, einen Lichtsensor, einen Tonsensor, ein Mikrofon oder einen Radarsensor enthalten. Dieser oder diese Sensoren sind mit einer Steuerung zum Ansteuern der UVC-Strahlungseinrichtung verbunden. Weiterhin kann die Steuerung derart ausgebildet sein, dass die UVC-Strahlungseinrichtung bei Leerfahrten der Aufzugskabine oder in Stillstandsphasen wenigstens temporär aktiviert wird.

Weiterhin ist es vorstellbar, die UVC-Strahlungseinrichtung zusätzlich zur Dekontamination der Kabinenluft einzusetzen. Die von der UVC-Strahlungseinrichtung erzeugten Strahlen können Viren oder andere Krankheitskeime in der Kabinenluft unschädlich zu machen.

Die UVC-Strahlungseinrichtung umfasst als Hohlspiegel ausgeführte Reflektoren, wobei vorzugsweise jeder Strahlungsquelle ein Reflektor zugeordnet ist. Somit weist die UVC-Strahlungseinrichtung mehrere nebeneinander angeordnete Reflektoren auf. Der Reflektor kann eine bevorzugte Grundform besitzen, die dazu eingerichtet ist, das von der zugehörigen UVC-LED ausgehende und auf den Reflektor auftreffende UVC-Strahlungsbündel zu kollimieren, und so im Wesentlichen parallel zu einer Abstrahlrichtung des Reflektors auszurichten. Der Reflektor ist derart ausgebildet und in der UVC-Strahlungseinrichtung angeordnet, dass die Abstrahlung des Reflektors schliesslich auf die Eingabeeinrichtung trifft. Der Reflektor kann bevorzugt derart ausgebildet sein, dass das Strahlenbündel nahezu in einer Rechteckform auf die Kabinenseitenwand auftrifft. Dieses Rechteck umgibt wenigstens einen Teilbereich der Oberfläche der Eingabeeinrichtung. Wenn mehrere Reflektoren vorgesehen sind, sind diese derart ausgebildet, dass sie zusammen die ganze Oberfläche der Eingabeeinrichtung abdecken. Auf diese Weise kann eine effiziente Bestrahlung der Eingabeeinrichtung sichergestellt werden. Insbesondere wird mit dieser Anordnung eine zielgenaue Bestrahlung ermöglicht. Neben der Eingabeeinrichtung können bei entsprechender Ausrichtung von gegebenenfalls zusätzlichen UVC-LEDs und zugehörigen Reflektoren auch Handläufe oder andere Oberflächen, die von Aufzugsnutzern mehr oder weniger regelmässig berührt werden, präzise zur Oberflächendesinfektion bestrahlt werden.

Die UVC-LEDs sind derart in der UVC-Strahlungseinrichtung angeordnet, dass die Abstrahlrichtung der Strahlungsquellen bzw. UVC-LEDs nach oben bzw. in Richtung der Kabinendecke gerichtet ist. Die derart ausgestaltete indirekte Bestrahlung hat den Vorteil, dass eine Fokussierung auf die gewünschte Fläche ermöglicht wird.

In einer besonders bevorzugten Ausführungsform ist die UVC-Strahlungseinrichtung hinsichtlich ihrer Aussenabmessungen niedrig bzw. schmal ausgeführt. Niedrig bedeutet hier, dass die vertikale Abmessung (Höhe) der UVC-Strahlungseinrichtung gegenüber einer der, vorzugsweise jeder der horizontalen Abmessungen (Länge, Breite) der UVC-Strahlungseinrichtung um ein mehrfaches, vorzugsweise wenigstens zweimal, besonders bevorzugt wenigstens dreimal grösser ist. Eine wenig gegenüber der Kabinendeckenoberfläche nach unten vorstehende UVC-Strahlungseinrichtung stellt ein optimales Raumangebot sicher. Ein weiterer Vorteil besteht darin, dass beispielsweise bei Umzügen oder Transport von grossen Gütern in der Aufzugskabine die UVC-Strahlungseinrichtung kaum ein Kollisionshindernis darstellt.

Die UVC-LEDs der UVC-Strahlungseinrichtung sind in einem Gehäuse verbaut, das an Decke angebracht ist. Eine solche UVC-Strahlungseinrichtung erlaubt ein einfaches Nachrüsten von Aufzugskabinen. Eine Montage der UVC-Strahlungseinrichtung an die Kabinendecke, so dass die UVC-Strahlungseinrichtung gegenüber der Kabinendeckenoberfläche nach unten bzw. in den Innenraum vorsteht ist allerdings nicht zwingend notwendig. Es wäre auch vorstellbar die UVC-Strahlungseinrichtung in der Decke zu integrieren und so wenigstens zum grössten Teil zu verstecken.

Die UVC-Strahlungseinrichtung kann ein Gehäuse mit einem einstückigen Gehäusebauteil umfassen, an dem Gehäusebauteil sowohl die UVC-LEDs als auch die Reflektoren befestigt sind. Das einstückige Gehäusebauteil kann zum Beispiel durch ein Aluminium-Strangprofil oder einem Spritzgussteil aus Kunststoff gebildet werden. Ein solches Gehäuse ist einfach und kostengünstig herstellbar.

Das Gehäuse für die UVC-Strahlungseinrichtung kann kastenförmig ausgestaltet sein. Beispielsweise könnte das Gehäuse ein einstückiges Gehäusebauteil mit einer C-Profilform umfassen. Das vorzugsweise nach unten oder alternativ zur Seite hin offene «C» weist eine offene Seite auf, die eine Strahlungsdurchlassöffnung für die von den UVC-LEDs generierten und gegebenenfalls von den Reflektoren umgelenkten UVC-Strahlen zum Bestrahlen der Eingabeeinrichtung bildet.

Eine besonders kompakte und platzsparende Anordnung ergibt sich, wenn das Gehäuse eine flächig an die Kabinendecke aufliegende Gehäuseoberseite und eine der Gehäuseoberseite gegenüberliegende, bevorzugt planparallel zur Gehäuseoberseite verlaufende Gehäuseunterseite aufweist. Das vorerwähnte einstückige Gehäusebauteil kann dabei die Gehäuseoberseite und die Gehäuseunterseite vorgeben.

Wenn die nebeneinander angeordneten UVC-LEDs auf einer Platine aufgebracht sind, ist erfindungsgemäss die Platine an einem Ende eines an der Gehäuseunterseite angeformten stegartigen Vorsprungs des Gehäuses angeordnet. Diese Anordnung zeichnet sich neben fertigungstechnischen Vorteilen dadurch aus, dass die von den LEDs in ihren Chips freigesetzte Wärme einfacher auf die Umgebung abgegeben werden kann.

Zusätzlich oder alternativ sind zum besseren Abführen der Wärme, die von den UVC-LEDs vor allem in ihren Chips und weiteren elektronischen Komponenten zum Betreiben der UVC-LEDs erzeugt wird, Kühlrippen am Gehäuse angeformt.

Gemäss dieser Variante weist das Gehäuse der UVC-Strahlungseinrichtung eine Gehäuseunterseite auf, die eine lamellenartige Struktur mit einer Mehrzahl von nach unten gerichteten Kühlrippen aufweist.

Weiterhin kann die UVC-Strahlungseinrichtung zum Verschliessen des Innenraums des Gehäuses eine transparente oder wenigstens UVC-Strahlung durchlässige Scheibe aufweisen, wodurch die UVC-LEDs vor Staub und anderen äusseren Einflüssen geschützt sind.

Die UVC-Strahlungseinrichtung kann mehrere Strahlungsquellen-Reihen mit UVC-LEDs aufweisen, die Reihen sind in horizontaler Richtung hintereinander angeordnet. Das Hintereinander bezieht sich auf die eingangs erwähnte Längsrichtung. Dadurch können auch vergleichsweise grossflächige Eingabeeinrichtungen effizient bestrahlt und so deren Oberflächen sterilisiert werden.

Alternativ zur vorher beschriebenen Unterbringung in einem Gehäuse ist es auch vorstellbar, die UVC-LEDs in einer offenen Trägerstruktur zu platzieren. Beispielsweise können hierzu die die oben erwähnten Strahlungsquellen-Reihen mit den UVC-LEDs an schrägen Flanken angeordnet sein, wobei die Flanken zur direkten Bestrahlung der Eingabeeinrichtung durch die Strahlungsquellen derart angeordnet sind, dass die Abstrahlrichtung der Strahlungsquellen auf die Eingabeeinrichtung zeigt bzw. gerichtet ist.

Als Trägerstruktur kann ein flächiges Profilbauteil mit einer Sägezahnprofilierung zum Bilden der Flanken zur Aufnahme der Strahlungsquellen-Reihen vorgesehen sein. Eine mit einem solchen Profilbauteil geschaffene UVC-Strahlungseinrichtung zeichnet sich durch eine besonders niedrige Bauhöhe aus.

Vorteilhaft kann es weiter sein, wenn die Strahlungsquelle neben der UVC-LED eine oder mehrere weitere LED mit unterschiedlichen Emissionsspektren aufweist, mit der oder denen unterschiedliche Farbmischungen erzeugbar sind. Die Strahlungsquelle kann in aktiviertem Zustand eine sichtbare Farbe, beispielsweise ein rotes Licht erzeugen, was als Warnung dient, dass gerade eine Desinfektion stattfindet.

Ein weiterer Aspekt der Erfindung kann sodann auf eine Montagehilfe (z.B. eine Montageschablone) gerichtet sein, wobei die Montagehilfe auf die Strahlungsrichtung der UVC-Strahlungseinrichtung abgestimmt ist und an der Eingabeeinrichtung angeschlagen werden kann und den Montageort für die UVC-Strahlungseinrichtung an der Decke anzeigt.

Weitere Einzelmerkmale und Vorteile der Erfindung ergeben sich aus der nachfolgenden Beschreibung von Ausführungsbeispielen und aus den Zeichnungen. Es zeigen:
- Fig. 1: eine stark vereinfachte Darstellung einer Aufzugskabine mit einem Kabinenbedienpanel und einer UVC-Strahlungseinrichtung,
- Fig. 1a: eine vergrösserte Darstellung der UVC-Strahlungseinrichtung zum Bestrahlen des Kabinenbedienpanels mit Strahlung im UVC-Bereich (Detail A aus Fig. 1),
- Fig. 2: eine vereinfachte Darstellung einer erfindungsgemässen Aufzugskabine mit einem Kabinenbedienpanel und einer UVC-Strahlungseinrichtung gemäss einem zweiten Ausführungsbeispiel,
- Fig. 3: eine Seitenansicht der UVC-Strahlungseinrichtung aus Fig. 2,
- Fig. 4: eine perspektivische Darstellung einer weiteren UVC-Strahlungseinrichtung,
- Fig. 5: in Doppelreihen angeordnete UVC-LEDs und zugehörige Reflektoren für eine UVC-Strahlungseinrichtung zum Bestrahlen des Kabinenbedienpanels mit Strahlung im UVC-Bereich in einer perspektivischen Darstellung, und
- Fig. 6: eine weitere UVC-Strahlungseinrichtung zum Bestrahlen des Kabinenbedienpanels mit Strahlung im UVC-Bereich in einer Seitenansicht.

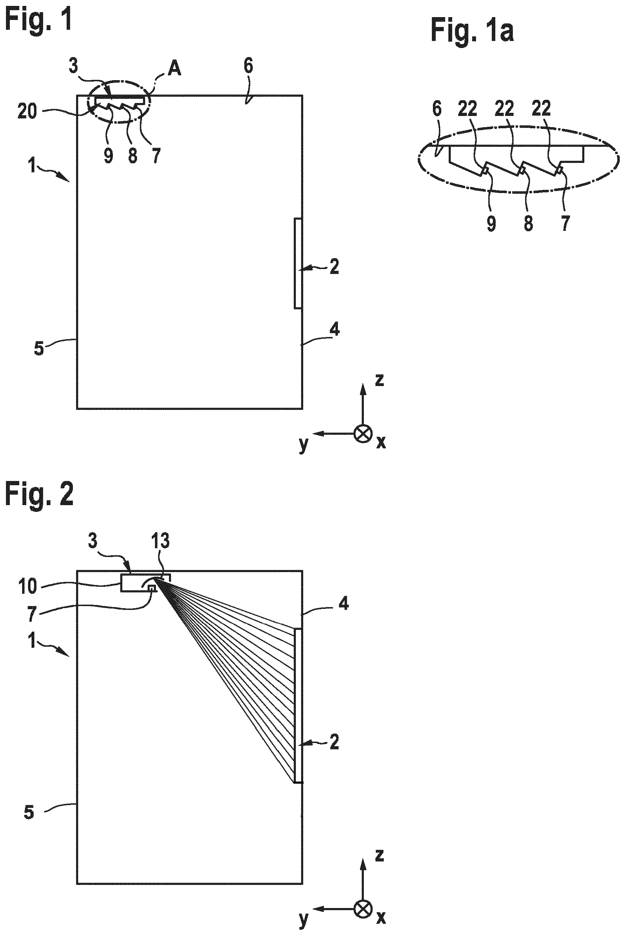

Fig. 1 zeigt eine mit 1 bezeichnete Aufzugskabine einer Aufzugsanlage für ein (hier nicht dargestelltes) mehrstöckiges Gebäude. Das Gebäude verfügt über einen Aufzugsschacht, in dem die Aufzugskabine 1 zum Transport von Personen oder Gütern zu einzelnen Stockwerken vertikal auf und ab bewegbar ist. Die Aufzugsanlage weist neben der Aufzugskabine 1 in der Regel ein (ebenfalls nicht dargestelltes) Gegengewicht und Tragmittel sowie einen Antrieb (z.B. ein Treibscheibenantrieb) auf. Der Antrieb treibt das oder die Tragmittel (z.B. Riemen, Stahlseile) an und bewegt damit die Aufzugskabine 1 und das Gegengewicht gegengleich.

Die Aufzugskabine 1 weist Kabinenseitenwände 4,5, eine Kabinendecke 6 und einen Kabinenboden auf, die zusammen einen Kabineninnenraum vorgegeben. Bei konventionellen Aufzugsanlagen ist in der Aufzugskabine 1 ein Kabinenbedienpanel 2 zum Eingeben eines Zielstockwerks angeordnet. Das Kabinenbedienpanel 2 ist dabei an der mit 4 bezeichneten ersten Kabinenseitenwand angebracht. Die der ersten Kabinenseitenwand 4 gegenüberliegende Kabinenseitenwand wird nachfolgend als zweite Kabinenseitenwand 5 bezeichnet. Der Abstand zwischen den beiden Kabinenseitenwänden 4 und 5 bemisst sich entlang einer Längsrichtung, die mit dem Pfeil y angedeutet ist. Unter Längsrichtung wird vorliegend mit anderen Worten die Richtung verstanden, die sich von der ersten, mit dem Kabinenbedienpanel 2 ausgerüsteten Kabinenseitenwand 4 zur gegenüberliegenden zweiten Kabinenseitenwand 5 hin erstreckt. Der Pfeil x definiert eine Querrichtung.

Zum Bestrahlen des Kabinenbedienpanels 2 mit Strahlung im UVC-Bereich weist die Aufzugskabine 1 eine UVC-Strahlungseinrichtung 3 zum Desinfizieren der von Aufzugsnutzern berührten Oberflächen des Kabinenbedienpanels auf. Die UVC-Strahlungseinrichtung 3 ist dabei ersichtlicherweise an der Kabinendecke 6 in einem hinteren Bereich in der Nähe der zweiten Kabinenseitenwand 5 angeordnet. Anstelle des hier beschriebenen Kabinenbedienpanels 2 könnten selbstverständlich auch andere Eingabeeinrichtungen zum Generieren eines Steuersignals an die Aufzugssteuerung mit der nachfolgend im Detail beschriebenen UVC-Strahlungseinrichtung 3 bestrahlt werden.

Die UVC-Strahlungseinrichtung 3 enthält als Strahlungsquellen UVC-LEDs 7, 8, 9. Die UVC-LEDs 7, 8, 9 sind bezüglich der Längsrichtung hintereinander angeordnet, wobei vorliegend die UVC-Strahlungseinrichtung 3 beispielhaft drei Reihen mit UVC-LEDs umfasst. Die vordersten UVC-LEDs sind die UVC-LEDs 7; die hintersten UVC-LEDs sind die UVC-LEDs 9. Die UVC-LEDs 7, 8, 9 sind an einer an der Kabinendecke befestigten Trägerstruktur 20 angeordnet. Diese Trägerstruktur 20 ist ein flächiges Profilbauteil mit einer Sägezahnprofilierung. Die Sägezahnprofilierung bildet mit 22 bezeichnete schräge Flanken. An den Flanken 22 sind die UVC-LEDs 7, 8, 9 angebracht. Die Flanken 22 sind zur Bestrahlung des Kabinenbedienpanel 2 durch die Strahlungsquellen bzw. UVC-LEDs 7, 8, 9 derart angeordnet, dass die Abstrahlrichtung der UVC-LEDs 7, 8, 9 auf das Kabinenbedienpanel 2 gerichtet ist. Die UVC-Strahlungseinrichtung 3 kann Bestandteil einer neuen Aufzugskabine 1 sein. Die hier beschriebenen UVC-Strahlungseinrichtungen 3 eignen sich aber auch zum Nachrüsten bereits bestehender Aufzugskabinen 1.

Die UVC-LEDs 7, 8, 9 emittieren, wenn sie aktiviert sind, eine Strahlung im UVC-Bereich zwischen 200 und 300nm. In diesem Bereich können unerwünschte Erreger wie Bakterien, Legionellen, Viren, Hefen und Pilze auf dem Kabinenbedienpanel 2 unschädlich gemacht werden. Bevorzugt emittieren die UVC-LEDs 7, 8, 9 Strahlung in einem Wellenlängenbereich zwischen 250nm und 280nm, in welchem eine besonders wirksame und zuverlässige Desinfektion der Kabinenbedienpanel-Oberflächen sichergestellt werden kann. Die von der UVC-Strahlungseinrichtung 3 abgegebene Strahlung im UVC-Bereich hat ausserdem einen reinigenden Effekt auf die Raumluft im Kabineninnenraum.

Die in Fig. 1 erkennbaren UVC-LEDs 7, 8, 9 sind gehören zu Strahlungsquellen-Reihen. Die jeweiligen Strahlungsquellen-Reihen bestehen aus einer Vielzahl von UVC-LEDs, die nebeneinander angeordnet sind und wobei sich die jeweiligen Reihen mit den nebeneinander angeordneten UVC-LEDs 7, 8, 9 sich in x-Richtung erstrecken.

Aus Fig. 1a ist entnehmbar, dass die jeweiligen Strahlungsquellen-Reihen mit den jeweiligen UVC-LEDs 7, 8, 9 an schrägen Flanken 21 angeordnet sind. Die Flanken 21 sind zur direkten Bestrahlung des Kabinenbedienpanels 2 durch die Strahlungsquellen derart angeordnet, dass die Abstrahlrichtung der Strahlungsquellen auf das Kabinenbedienpanel 2 gerichtet ist. Die offene Trägerstruktur 20 ist als flächiges Profilbauteil mit einer Sägezahnprofilierung ausgestaltet. Die Sägezahnprofilierung bildet die Flanken 22 zur Aufnahme der Strahlungsquellen-Reihen mit den UVC-LEDs 7, 8, 9.

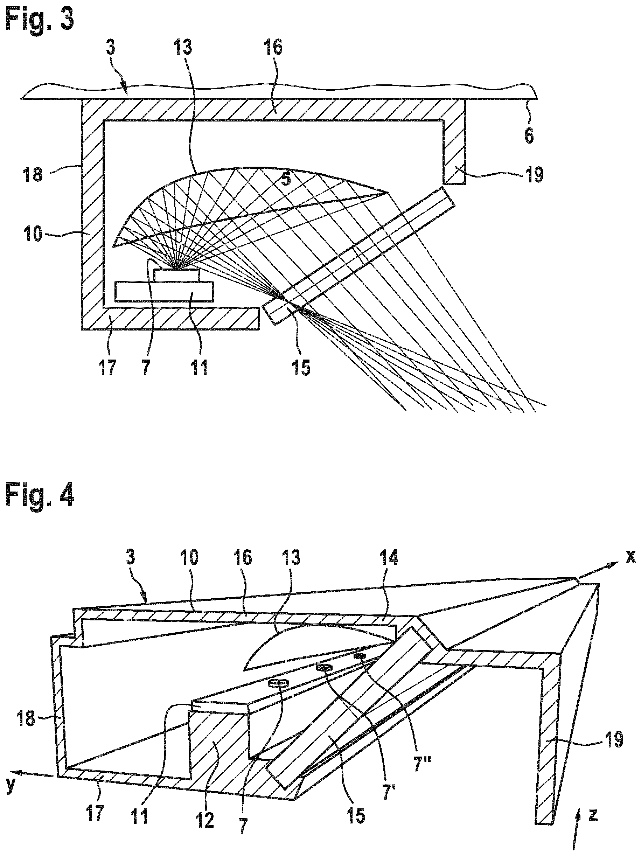

Fig. 2 betrifft ein zweites Ausführungsbeispiel einer Aufzugskabine 1 mit einer Eingabeeinrichtung 2 zum Generieren eines Steuersignals an die Aufzugssteuerung, wie etwa das vorhin erwähnte Kabinenbedienpanel zum Eingeben eines Zielstockwerks und einer UVC-Strahlungseinrichtung 3 zum Bestrahlen der Eingabeeinrichtung 2 mit Strahlung im UVC-Bereich zum Desinfizieren der von Aufzugsnutzern berührten Oberflächen der Eingabeeinrichtung 2. Die UVC-Strahlungseinrichtung 3 enthält UVC-LEDs 7 als Strahlungsquellen, die in einem Gehäuse 10 verbaut sind. Das Gehäuse 10 der UVC-Strahlungseinrichtung 3 ist an der Kabinendecke 6 angebracht. Die UVC-LEDs 7 sind derart in der UVC-Strahlungseinrichtung 3 angeordnet, dass die Abstrahlrichtung der Strahlungsquellen bzw. UVC-LEDs nach oben gerichtet ist. Die UVC-Strahlungseinrichtung 3 verfügt weiter wenigstens einen als Hohlspiegel ausgeführten Reflektor 13. Der Reflektor 13 ist derart ausgebildet, dass die Abstrahlung der Reflektoren 13 auf die Eingabeeinrichtung trifft. Die Abstrahlrichtungen vom Reflektor 13 sind in der Seitenansicht gemäss Fig. 2 durch fächerartig sich ausbreitende Linien angedeutet; in der nachfolgenden Fig. 3 sind die jeweils durch einzelne Linien angedeutete Strahlrichtungen der UVC-Strahlung der UVC-Strahlungseinrichtung 3 noch besser erkennbar.

Die UVC-Strahlungseinrichtung 3 weist ein kastenförmig ausgestaltetes Gehäuse 10 auf. Das Gehäuse 10 umfasst eine flächig an die Kabinendecke aufliegende Gehäuseoberseite 16 und eine der Gehäuseoberseite gegenüberliegende, bevorzugt planparallel zur Gehäuseoberseite verlaufende Gehäuseunterseite 17, sowie rechtwinklig zur Gehäuseoberseite 16 und Gehäuseunterseite 17 verlaufende Gehäuseseitenwände 18, 19. Zum Verschliessen des Innenraums des Gehäuses 10 ist eine transparente für UVC-Strahlung durchlässige Scheibe 15 im Bereich der vorderen Gehäuseseitenwand 19 vorgesehen. Die UVC-LEDs 7 sind auf einer Platine 11 aufgebracht, die an der Gehäuseunterseite 17 fixiert ist.

Konstruktive Details für eine mögliche Ausgestaltung der UVC-Strahlungseinrichtung 3 sind in Fig. 4 gezeigt. Das Gehäuse 10 umfasst ein einstückiges Gehäusebauteil 14, an dem sowohl die UVC-LEDs 7 als auch die Reflektoren 13 befestigt sind. Das Gehäusebauteil 14 kann zum Beispiel durch ein Aluminium-Strangpressprofil gebildet werden.

Aus Figur 4 ist sodann erkennbar, dass die UVC-LEDs 7 eine Reihe bilden, die in x-Richtung verläuft. Jede Reihe umfasst mehrere voneinander beabstandete UVC-LEDs, die nebeneinander angeordnet sind. In der ersten Strahlungsquellen-Reihe sind die einzelnen UVC-LEDs bzw. Strahlungsquellen mit 7, 7', 7" bezeichnet. Jeder UVC-LED 7, 7', 7" ist dabei ein Reflektor 13 zugeordnet.

Die nebeneinander angeordneten UVC-LEDs 7, 7', 7" sind auf einer Platine 11 aufgebracht. An der Gehäuseunterseite 17 ist ein stegartiger Vorsprung 12 angeformt. Die Platine 11 ist vorliegend am oberen Ende dieses stegartigen Vorsprungs 12 angeordnet.

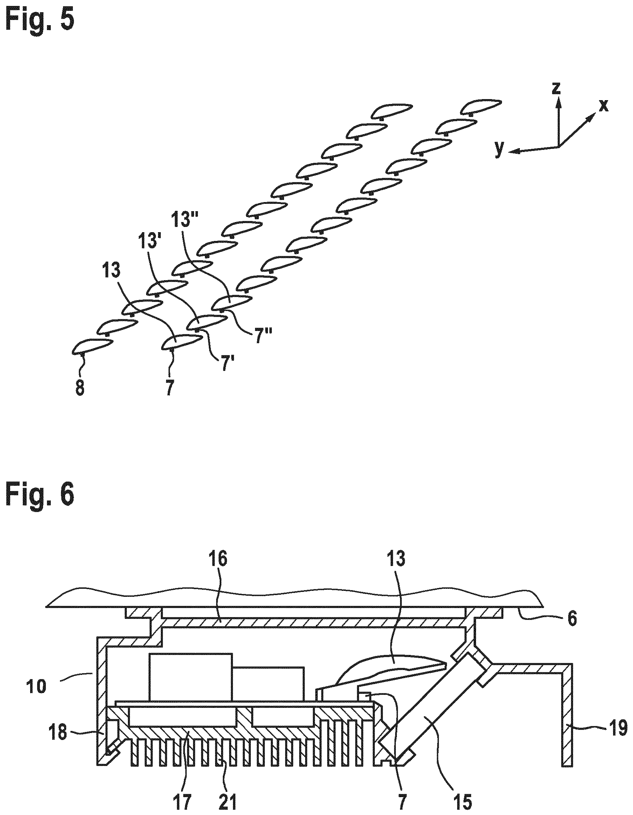

Je nach Grösse der anzustrahlenden Oberfläche der Eingabeeinrichtung 2 kann es notwendig sein, mehr als eine Strahlungsquellen-Reihe in der UVC-Strahlungseinrichtung 3 anzuordnen. Eine solche Anordnung ist in Fig. 5 gezeigt, wobei zum besseren Verständnis lediglich die Strahlungsquellen und Reflektoren in Fig. 5 dargestellt sind. Nicht dargestellt ist das Gehäuse, in dem die Strahlungsquellen und Reflektoren untergebracht sind. Die hier gezeigte Anordnung betrifft eine Doppelreihe aus Strahlungsquellen und Reflektoren. Die erste Reihe umfasst die nebeneinander angeordneten UVC-LEDs 7, 7', 7", etc. Die nebeneinander angeordneten UVC-LEDs 7, 7', 7" sind in regelmässigen Abständen aneinander gereiht, wobei der Abstand zwischen zwei UVC-LEDs mindestens 5 cm betragen kann. Die zugehörigen als Hohlspiegel ausgebildeten Reflektoren sind mit 13, 13', 13" etc. bezeichnet. Die zweite Reihe mit den UVC-LEDs 8 ist gleichartig ausgestaltet.

Fig. 6 zeigt eine weitere UVC-Strahlungseinrichtung 3 zum Bestrahlen der Eingabeeinrichtung 2 mit Strahlung im UVC-Bereich zum Desinfizieren der von Aufzugsnutzern berührten Oberflächen. Diese UVC-Strahlungseinrichtung 3 unterscheidet sich von der in Fig. 4 gezeigten UVC-Strahlungseinrichtung im Wesentlichen durch eine andere Gestaltung des Gehäuses 10. Hier ist das Gehäuse 10 mehrteilig ausgestaltet. Zum Abführen der Wärme, die von den UVC-LEDs 7 in ihren Chips und weiteren elektronischen Komponenten zum Betreiben der UVC-LEDs 7 erzeugt wird, sind am Gehäuse 10 Kühlrippen 21 angeformt. Das Gehäuse 10 weist hierzu eine Gehäuseunterseite 17 mit einer lamellenartigen Struktur auf. Diese lamellenartige Struktur wird durch mehrere nach unten gerichtete Kühlrippen 21 gebildet.

## Patentansprüche

1. Aufzugskabine (1) mit wenigstens einer an einer ersten Kabinenseitenwand (4) angeordneten Eingabeeinrichtung (2) und einer UVC-Strahlungseinrichtung (3) zum Bestrahlen der Eingabeeinrichtung (2) mit Strahlung im UVC-Bereich, wobei die UVC-Strahlungseinrichtung (3) an der Kabinendecke (6) angeordnet ist, und wobei die UVC-Strahlungseinrichtung (3) eine Vielzahl von in horizontaler Richtung nebeneinander angeordneten UVC-LEDs (7,7',7") als Strahlungsquellen umfasst, wobei die UVC-LEDs (7,7',7") in einem vorzugsweise kastenförmig ausgestalteten Gehäuse (10) verbaut sind, das an der Kabinendecke (6) angebracht ist, **dadurch gekennzeichnet, dass** die UVC-Strahlungseinrichtung (3) als Hohlspiegel ausgeführte Reflektoren (13,13',13") umfassen, wobei vorzugsweise jeder Strahlungsquelle ein Reflektor (13,13',13") zugeordnet ist, wobei die UVC-LEDs (7,7',7") derart in der UVC-Strahlungseinrichtung (3) angeordnet oder anordbar sind, dass deren Abstrahlrichtung nach oben gerichtet ist, dass die nebeneinander angeordneten UVC-LEDs (7,7',7") auf einer Platine (11) aufgebracht sind und dass die Platine (11) an einem Ende eines an einer Gehäuseunterseite (17) angeformten stegartigen Vorsprungs (12) des Gehäuses (10) angeordnet ist und/oder dass das Gehäuse (10) eine Gehäuseunterseite (17) aufweist, die eine lamellenartige Struktur mit einer Mehrzahl von nach unten gerichteten Kühlrippen (21) aufweist.

2. Aufzugskabine (1) nach Anspruch 1, **dadurch gekennzeichnet, dass** das Gehäuse (10) ein einstückiges Gehäusebauteil (14) umfasst, wobei am Gehäusebauteil (14) sowohl die UVC-LEDs (7,7',7") als auch die Reflektoren (13,13',13") befestigt sind.

3. Aufzugskabine (1) nach Anspruch 1 oder 2, **dadurch gekennzeichnet, dass** das Gehäuse (10) eine flächig an die Kabinendecke (6) aufliegende Gehäuseoberseite (16) aufweist und dass die der Gehäuseoberseite gegenüberliegende, bevorzugt planparallel zur Gehäuseoberseite Gehäuseunterseite (17) verläuft.

4. Aufzugskabine (1) nach einem der Ansprüche 1 bis 3, **dadurch gekennzeichnet, dass** die UVC-Strahlungseinrichtung (3) zum Verschliessen des Innenraums des kastenförmigen Gehäuses (10) eine Scheibe (15) aufweist.

5. Aufzugskabine (1) nach einem der Ansprüche 1 bis 4, **dadurch gekennzeichnet, dass** die UVC-Strahlungseinrichtung (3) wenigstens zwei hintereinander angeordnete Strahlungsquellen-Reihen mit UVC-LEDs (7,7',7"; 8; 9) aufweist.

6. Aufzugskabine (1) nach Anspruch 1 und Anspruch 5, **dadurch gekennzeichnet, dass** die jeweiligen Strahlungsquellen-Reihen mit den UVC-LEDs (7,7',7"; 8; 9) an schrägen Flanken (22) angeordnet sind, wobei die Flanken (22) zur direkten Bestrahlung der Eingabeeinrichtung (2) derart angeordnet sind, dass die Abstrahlrichtung der Strahlungsquellen auf die Eingabeeinrichtung (2) gerichtet ist.

7. Aufzugsanlage nach Anspruch 6, **dadurch gekennzeichnet, dass** als Trägerstruktur für die UVC-LEDs (7,7',7"; 8; 9) ein flächiges Profilbauteil (20) mit einer Sägezahnprofilierung zum Bilden der Flanken (22) zur Aufnahme der Strahlungsquellen-Reihen mit den UVC-LEDs (7,7',7"; 8; 9) vorgesehen ist.

8. Aufzugskabine (1) nach einem der Ansprüche 1 bis 7, **dadurch gekennzeichnet, dass** wenigstens einer der Strahlungsquellen UVC-Strahlungseinrichtung (3) neben der UVC-LED (7,7',7"; 8; 9) eine oder mehrere weitere LED mit unterschiedlichen Emissionsspektren aufweist, mit der unterschiedliche Farbmischungen erzeugbar sind.

## Claims

1. An elevator car (1) comprising at least one input device (2) arranged on a first side wall (4) of the car and a UVC radiation device (3) for irradiating the input device (2) with radiation in the UVC area, wherein the UVC radiation device (3) is arranged on the car ceiling (6), and wherein the UVC irradiation device (3) comprises a plurality of UVC LEDs (7, 7', 7'') arranged side by side in the horizontal direction ) as radiation sources , wherein the UVC LEDs (7, 7', 7'') are installed in a housing (10) preferably designed in the shape of a box, which is mounted on the cabin ceiling (6), **characterized in that** the UVC radiation device (3) comprises reflectors (13, 13', 13") designed as parabolic mirrors, wherein preferably one reflector (13, 13', 13") is assigned to each radiation source, and wherein the UVC LEDs (7, 7', 7") are arranged or can be arranged within the UVC radiation device (3) such that their radiation direction is directed upward, that the UVC LEDs (7, 7', 7'') arranged side by side are mounted on a circuit board (11), and that the circuit board (11) is arranged at one end of a web-like projection (12) of the housing (10) formed on a bottom surface (17) of the housing, and/or that the housing (10) has a bottom surface (17) that features a lamellar structure with a plurality of downward-facing cooling fins (21).

2. Elevator car (1) according to claim 1, **characterized in that** the housing (10) comprises a one-piece housing component (14), wherein both the UVC LEDs (7, 7', 7'') and the reflectors (13, 13', 13'') are mounted on the housing component (14).

3. Elevator car (1) according to claim 1 or 2, **characterized in that** the housing (10) has a top surface (16) that rests flat against the car ceiling (6), and that the bottom surface (17) of the housing, which is opposite the top surface and preferably plane-parallel to it, extends.

4. Elevator car (1) according to any one of claims 1 through 3, **characterized in that** the UVC radiation device (3) comprises a pane (15) for sealing off the interior of the box-shaped housing (10).

5. Elevator car (1) according to any one of claims 1 through 4, **characterized in that** the UVC radiation device (3) comprises at least two rows of radiation sources arranged one behind the other comprising UVC LEDs (7, 7', 7''; 8; 9).

6. Elevator car (1) according to Claim 1 and Claim 5, **characterized in that** the respective rows of radiation sources comprising the UVC LEDs (7, 7', 7''; 8; 9) are arranged on slanted flanks (22), wherein the flanks (22) are arranged for direct irradiation of the input device (2) such that the radiation direction of the radiation sources is directed toward the input device (2).

7. Elevator installation according to claim 6, **characterized in that** a flat profile component (20) with a sawtooth profile is used as the support structure for the UVC LEDs (7, 7', 7"; 8; 9), a flat profiled component (20) with a sawtooth profile is provided as a support structure to form the flanks (22) for accommodating the rows of radiation sources comprising the UVC LEDs (7, 7', 7''; 8; 9).

8. An elevator car (1) according to any one of claims 1 through 7, **characterized in that** at least one of the radiation source UVC radiation devices (3) comprises, in addition to the UVC LED (7, 7', 7''; 8; 9), one or more additional LEDs with different emission spectra, with which different color mixtures can be produced.

## Revendications

1. Cabine d'ascenseur (1) comprenant au moins un dispositif d'entrée (2) disposé sur une première paroi latérale (4) de la cabine et un dispositif d'irradiation UVC (3) destiné à irradier le dispositif d'entrée (2) avec un rayonnement dans la zone UVC, le dispositif d'irradiation UVC (3) est disposé au plafond de la cabine (6), et dans laquelle le dispositif d'irradiation UVC (3) comprend une pluralité de LED UVC ( 7,7',7'' disposées côte à côte dans la direction horizontale ) servant de sources de rayonnement , les LED UVC (7, 7', 7") étant intégrées dans un boîtier (10) de préférence en forme de caisson, qui est fixé au plafond de la cabine (6), **caractérisé en ce que** le dispositif de rayonnement UVC (3) comprend des réflecteurs (13, 13', 13") réalisés sous forme de miroirs concaves, un réflecteur (13, 13', 13") étant de préférence associé à chaque source de rayonnement, les LED UVC (7, 7', 7") sont ou peuvent être disposées dans le dispositif de rayonnement UVC (3) de telle sorte que leur direction de rayonnement soit orientée vers le haut, que les LED UVC (7, 7', 7") disposées côte à côte soient montées sur une carte (11) et que la carte (11) est disposée à une extrémité d'une saillie en forme de nervure (12) du boîtier (10), formée sur une face inférieure (17) de celui-ci, et/ou **en ce que** le boîtier (10) présente une face inférieure (17) qui comporte une structure en lamelles avec une pluralité d'ailettes de refroidissement (21) orientées vers le bas.

2. Cabine d'ascenseur (1) selon la revendication 1, **caractérisée en ce que** le boîtier (10) comprend un élément de boîtier monobloc (14), les LED UVC (7, 7', 7") ainsi que les réflecteurs (13, 13', 13") sont fixés sur l'élément de boîtier (14).

3. Cabine d'ascenseur (1) selon la revendication 1 ou 2, **caractérisée en ce que** le boîtier (10) présente une face supérieure (16) reposant à plat contre le plafond (6) de la cabine et **en ce que** la face inférieure (17) du boîtier, située à l'opposé de la face supérieure et de préférence plane et parallèle à celle-ci, s'étend .

4. Cabine d'ascenseur (1) selon l'une des revendications 1 à 3, **caractérisée en ce que** le dispositif d'irradiation UVC (3) comporte une vitre (15) pour fermer l'espace intérieur du boîtier en forme de caisson (10) .

5. Cabine d'ascenseur (1) selon l'une des revendications 1 à 4, **caractérisée en ce que** le dispositif de rayonnement UVC (3) comporte au moins deux rangées de sources de rayonnement disposées l'une derrière l'autre comprenant des LED UVC (7, 7', 7'' ; 8 ; 9).

6. Cabine d'ascenseur (1) selon la revendication 1 et la revendication 5, **caractérisée en ce que** les rangées de sources de rayonnement respectives comprenant les LED UVC (7, 7', 7'' ; 8 ; 9) sont disposées sur des flancs inclinés (22), les flancs (22) étant agencés pour irradier directement le dispositif d'entrée (2) de telle sorte que la direction de rayonnement des sources de rayonnement soit orientée vers le dispositif d'entrée (2) .

7. Installation d'ascenseur selon la revendication 6, **caractérisée en ce que**, en tant que structure de support pour les LED UVC (7, 7', 7'' ; 8 ; 9) , il est prévu un élément profilé plat (20) présentant un profil en dents de scie pour former les flancs (22) destinés à recevoir les rangées de sources de rayonnement comprenant les LED UVC (7, 7', 7'' ; 8 ; 9) .

8. Cabine d'ascenseur (1) selon l'une des revendications 1 à 7, **caractérisée en ce qu'**au moins l'un des dispositifs de rayonnement UVC (3) comprend, outre la LED UVC (7, 7', 7'' ; 8 ; 9), une ou plusieurs autres LED présentant des spectres d'émission différents, permettant de produire différents mélanges de couleurs.
